# EUROPEAN PATENT APPLICATION

(11) **EP 1 760 064 A1**
(43) Date of publication of application: **07.03.2007**
(21) Application number: 05750962.2
(22) Date of filing: 20.06.2005
(51) Int. Cl.: C07C 51/347, C07B 59/00, C07C 57/54, C07B 61/00

(54) **METHOD FOR DEUTERATING HALOACRYLIC ACID OR ITS SALT**

(30) Priority: 25.06.2004 JP 2004187152
(71) Applicant: Wako Pure Chemical Industries, Ltd., Osaka-shi, Osaka 540-8605 (JP)
(72) Inventor: MAESAWA, Tsuneaki, 3501101 (JP); ITO, Nobuhiro, 3501101 (JP); HIROTA, Kosaku, Nagoya-shi, Aichi 4640031 (JP); SAJIKI, Hironao, 5020823 (JP)
(74) Representative: Kaiser, Jürgen
(86) International application number: PCT/JP2005/011228
(87) International publication number: WO 2006/001236

(57) **Abstract**

The object of the present invention is to provide a method for efficiently and industrially deuterating a haloacrylic acid or salt thereof. The present invention is an invention of a deuteration method of a compound represented by the general formula [1]: 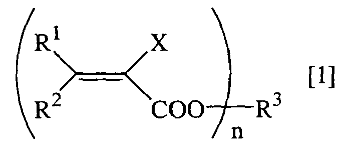 wherein, R¹ and R² indicate each independently a light hydrogen atom or a heavy hydrogen atom and at least one of R¹ and R² indicates a light hydrogen atom; R³ indicates a light hydrogen atom, a heavy hydrogen atom, an alkaline metal atom or an alkaline earth metal atom; X indicates a halogen atom; and n indicates 1 or 2,
which comprises reacting the compound represented by the general formula [1] with a heavy hydrogen source in the presence of a catalyst selected from a palladium catalyst, a platinum catalyst, a rhodium catalyst, a ruthenium catalyst, a nickel catalyst and a cobalt catalyst that are not subjected to activation processing; and an invention of a deuterated compound represented by the general formula [2]: 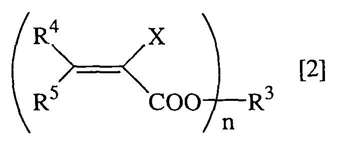 wherein, R⁴ and R⁵ indicate each independently a light hydrogen atom or a heavy hydrogen atom and at least one of R⁴ and R⁵ indicates a heavy hydrogen atom; R³ indicates a light hydrogen atom, a heavy hydrogen atom, an alkaline metal atom or an alkaline earth metal atom; X indicates a halogen atom; and n indicates 1 or 2.

## Description

### TECHNICAL FIELD

The present invention relates to a deuteration method of a haloacrylic acid or salt thereof, which is carried out using a catalyst.

### BACKGROUND ART

It is said that a deuterated (deuterated and tritiated) compound is useful for various purposes. A deuterated compound, for example, is very useful in investigating a reaction mechanism, substance metabolism and is broadly used as a labeled compound. It is further said that the compound is also useful as a pharmaceutical, an agrichemical, an organic EL material because the stability and properties of the compound itself vary according to an isotope effect. It is also said that a tritiated compound is useful as a labeled compound in animal experiments to investigate absorption, distribution, concentration in blood, excretion, metabolism of pharmaceuticals. By these reasons, researches have been lately prevalent in these fields using a deuterated (deuterated and tritiated) compound.

Recent interest has focused on a deuterated haloacrylic acid or salt thereof as an intermediate of a monomer for an optical fiber.

Various methods are available to obtain such deuterated compounds. Among these, however, a technology for deuterating a carboxylic acid or salt thereof having a carbonyl group or a hydroxyl group in the structure is still so immature that it has been difficult to efficiently and industrially obtain the deuterated compound.

Conventional technologies include, for example, 1) a method for deuterating a carboxylic acid under a basic condition using heavy hydrogen peroxide (see Patent Literature 1), 2) a method for deuterating an alcohol or a carboxylic acid using an iridium complex as a catalyst and deuterated water as a heavy hydrogen source (see Non-Patent Literature 1), 3) a method for deuterating an aliphatic acid using a palladium carbon as a catalyst and only heavy hydrogen gas as a heavy hydrogen source (see Non-Patent Literature 2), 4) a method for deuterating acrylic acid, methyl acrylate, methacrylic acid and methyl methacrylate using a metal selected from the group 8 metals as a catalyst and using deuterated water or a combination of deuterated water and heavy hydrogen gas as a heavy hydrogen source (see Patent Literature 2, Patent Literature 3 and Patent Literature 4), or 5) a method for deuterating acrylic acid and methyl methacrylate using a catalyst not activated by hydrogen and using deuterated water as a heavy hydrogen source (see Patent Literature 5).

These methods, however, do not describe anything about a method for deuterating a haloacrylic acid or salt thereof as a substrate. Each method has a problem shown below.

1) The method for deuterating a carboxylic acid using heavy hydrogen peroxide under a basic condition has the problem that the reaction solution is not neutral and needs a complicated purifying operation to isolate a compound deuterated by the method.

2) The method for deuterating an alcohol or a carboxylic acid using an iridium complex not subjected to activation processing as a catalyst and using deuterated water as a heavy hydrogen source has the problems that the deuteration ratio gets high for the hydrogen atoms remote from the carbon atom bonded to the hydroxyl group of the carboxylic acid, whereas the deuteration ratio is extremely low for the hydrogen atoms close to the hydroxyl group, and that the iridium complex itself to be used as a catalyst is an unstable compound and difficult to produce or acquire.

3) The method for deuterating an aliphatic acid using as a catalyst a palladium carbon not subjected to activation processing and using as a heavy hydrogen source heavy hydrogen gas generated in electrolysis of KOD + D₂O has the problems that a special device is necessary for preparing the heavy hydrogen gas and is not practical due to cumbersome operation and that it is difficult to deuterate a compound such as an unsaturated aliphatic acid having an unsaturated bond vulnerable to reduction by hydrogenation.

4) The method for deuterating acrylic acid, methyl acrylate, methacrylic acid or methyl methacrylate using a metal selected from the metals of the group 8 not subjected to activation processing as a catalyst and using deuterated water or a combination of deuterated water and heavy hydrogen gas as a heavy hydrogen source has the problem that the deuteration ratio is low in the case where only deuterated water is used as a heavy hydrogen source, whereas in the case where a combination of deuterated water and heavy hydrogen gas is used as a heavy hydrogen source, a carbon - carbon double bond in acrylic acid, methyl acrylate, methacrylic acid or methyl methacrylate, which is a reactive substrate, tends to be hydrogenated (catalytic reduction) in the deuteration reaction and thus deuteration is hardly carried out keeping the double bond as it is.

5) The method for deuterating acrylic acid and methyl methacrylate using as a catalyst and a catalyst not subjected to activation processing using deuterated water as a heavy hydrogen source has the problem that the deuteration ratio is low.

In the above situations, development of a method for efficiently and industrially deuterating a haloacrylic acid or salt thereof as a substrate has been desired.

Patent Literature 1: U.S. Pat. No. 3,849,458
Patent Literature 2: JP-B-5-19536
Patent Literature 3: JP-A-61-277648
Patent Literature 4: JP-A-61-275241
Patent Literature 5: JP-A-63-198638
Non-Patent Literature 1: J. Am. Chem. Soc. Vol. 124, No. 10, 2092 (2002)
Non-Patent Literature 2: LIPIDS, Vol. 9, No. 11, 913 (1974)

### DISCLOSURE OF THE INVENTION

### PROBLEM TO BE SOLVED BY THE INVENTION

Considering the above situations, it is an object of the present invention to provide a method for efficiently and industrially deuterating a haloacrylic acid or salt thereof.

### MEANS TO SOLVE THE SUBJECT

The present invention is an invention of a deuteration method of a compound represented by the general formula [1]:

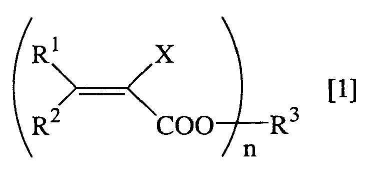

wherein, R¹ and R² indicate each independently a light hydrogen atom or a heavy hydrogen atom and at least one of R¹ and R² indicates a light hydrogen atom; R³ indicates a light hydrogen atom, a heavy hydrogen atom, an alkaline metal atom or an alkaline earth metal atom; X indicates a halogen atom; and n indicates 1 or 2,
which comprises reacting the compound represented by the general formula [1] with a heavy hydrogen source in the presence of a catalyst selected from a palladium catalyst, a platinum catalyst, a rhodium catalyst, a ruthenium catalyst, a nickel catalyst and a cobalt catalyst that are not subjected to activation processing; and an invention of a deuterated compound represented by the general formula [2]:

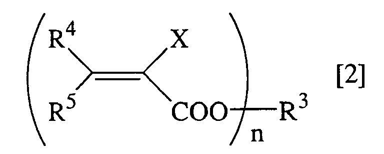

wherein, R⁴ and R⁵ indicate each independently a light hydrogen atom or a heavy hydrogen atom and at least one of R⁴ and R⁵ indicates a heavy hydrogen atom; R³ indicates a light hydrogen atom, a heavy hydrogen atom, an alkaline metal atom or an alkaline earth metal atom; X indicates a halogen atom; and n indicates 1 or 2.

### EFFECT OF THE INVENTION

Unlike conventional methods carried out under severe conditions such as a basic condition, the deuteration (deuteration and tritiation) method of the present invention can be carried out under a neutral condition by reacting a compound represented by the general formula [1] with a heavy hydrogen source in the presence of a catalyst not subjected to activation processing and thus remarkably improves work environments. Further, the deuteration method of the present invention enables a compound represented by the general formula [1], having a carbon - carbon double bond to be efficiently deuterated without reducing the double bond by hydrogenation and also without causing reductive elimination of a halogen present in the compound. As deuteration of an acrylic acid or ester thereof using a catalyst not subjected to activation processing in conventional technologies has resulted in a low deuteration ratio, it has been forecasted that deuteration of a haloacrylic acid having a similar structure to an acrylic acid using a similar method will end in a low deuteration ratio and a halogen atom in the haloacrylic acid will suffer reductive elimination in the process of deuteration. Therefore, it was wholly beyond expectation to get such a good result.

### BEST MODE FOR CARRYING OUT THE INVENTION

In the present invention, a heavy hydrogen atom means a deuterium (D) atom or a tritium (T) atom and deuteration means substitution with deuterium and tritium. Further, in the present specification, deuteration ratio means the ratio of the amount of hydrogen atoms substituted with heavy hydrogen atoms to the total hydrogen atoms contained in a compound represented by the general formula [1]. Furthermore, the "catalyst not subjected to activation processing" means a catalyst that is not subjected to activation processing such as being brought into contact with hydrogen gas or heavy hydrogen gas after prepared by an ordinary method.

In the deuteration method of the present invention, the halogen atom indicated by X in a compound represented by the general formula [1] includes, for example, a fluorine atom, a chlorine atom, a bromine atom and an iodine atom, among these, for example, a fluorine atom, a chlorine atom and a bromine atom are preferable, in particular, a chlorine atom is more preferable.

The alkaline metal atom indicated by R³ includes, for example, a lithium atom, a sodium atom, a potassium atom, a rubidium atom and a cesium atom, among these, a sodium atom or a potassium atom is preferable, in particular, a sodium atom is more preferable.

The alkaline earth metal atom indicated by R³ includes, for example, a magnesium atom, a calcium atom, strontium atom and a barium atom, among these, a magnesium atom or a calcium atom is preferable.

Preferable specific examples of a compound represented by the general formula [1] include, for example, 2-fluoroacrylic acid, 2-chloroacrylic acid, 2-bromoacrylic acid, 2-iodoacrylic acid and salts thereof (e.g., an alkaline metal salt such as a sodium salt, a potassium salt and a rubidium salt; an alkaline earth metal salt such as a magnesium salt, a calcium salt, a strontium salt and a barium salt), among these, for example, a 2-fluoroacrylate (e.g., a sodium salt, a potassium salt etc.), a 2-chloroacrylate (e.g., a sodium salt, a potassium salt etc.), or a 2-bromoacrylate (e.g., a sodium salt, a potassium salt etc.) is preferable, in particular, sodium 2-chloroacrylate or potassium 2-chloroacrylate is more preferable.

A compound represented by the general formula [1] may be obtained on the market or synthesized as appropriate by an ordinary method.

A heavy hydrogen source to be reacted with the above compounds represented by the general formula [1] in the deuteration method of the present invention includes, for example, a deuterated solvent.

The deuterated solvent to be used as a heavy hydrogen source includes, in the case where the heavy hydrogen is a deuterium, for example, deuterated water (D₂O); deuterated alcohols such as deuterated methanol, deuterated ethanol, deuterated isopropanol, deuterated butanol, deuterated tert-butanol, deuterated pentanol, deuterated hexanol, deuterated heptanol, deuterated octanol, deuterated nonanol, deuterated decanol, deuterated undecanol and deuterated dodecanol; deuterated carboxylic acids such as deuterated formic acid, deuterated acetic acid, deuterated propionic acid, deuterated butyric acid, deuterated isobutyric acid, deuterated valeric acid, deuterated isovaleric acid and deuterated pivalic acid; deuterated ketones such as deuterated acetone, deuterated methyl ethyl ketone, deuterated methyl isobutyl ketone, deuterated diethyl ketone, deuterated dipropyl ketone, deuterated diisopropyl ketone and deuterated dibutyl ketone; and organic solvents such as deuterated dimethyl sulfoxide. Among these, deuterated water and deuterated alcohols are preferable, and specifically deuterated water and deuterated methanol are particularly preferable. In addition, deuterated water is preferable in view of ecology and workability. In the case where the heavy hydrogen is a tritium, the deuterated solvent to be used as a heavy hydrogen source includes, for example, tritiated water (T₂O).

The deuterated solvent may be one wherein at least one hydrogen atom in the molecule is deuterated, and for example, deuterated alcohols wherein at least a hydrogen atom in a hydroxyl group is deuterated, or deuterated carboxylic acids wherein at least a hydrogen atom in a carboxyl group is deuterated, can be used in a method for deuteration of the present invention, and among others, a solvent wherein all hydrogen atoms in the molecule are deuterated is more preferable.

As an amount of a heavy hydrogen source to be used increases, deuteration of the present invention tends to proceed further. In view of cost, however, an amount of heavy hydrogen atoms contained in the heavy hydrogen source is such level, as lower limit, of preferably in the order of not less than equimolar, 10 molar times, 20 molar times, 30 molar times and 40 molar times, and as upper limit, of preferably in the order of 250 molar times and 150 molar times, based on hydrogen atoms deuteratable in a compound, based on the amount of the deuteratable hydrogen atom in a compound represented by the general formula [1] as a reactive substrate.

A reaction solvent may be used as necessary in the deuteration method of the present invention. Because a reaction system of deuteration of the present invention may be in a suspended state, a solvent that hardly dissolves a substrate can be used as the reaction solvent, but a solvent that easily dissolves the substrate is preferable.

Specific examples of the reaction solvent to be used as necessary include, for example, ethers such as dimethyl ether, diethyl ether, diisopropyl ether, ethylmethyl ether, tert-butylmethyl ether, 1,2-dimethoxyethane, oxirane, 1,4-dioxane, dihydropyrane and tetrahydrofuran; aliphatic hydrocarbons such as hexane, heptane, octane, nonane, decane and cyclohexane; alcohols such as methanol, ethanol, isopropanol, butanol, tert-butanol, pentanol, hexanol, heptanol, octanol, nonanol, decanol, undecanol and dodecanol; carboxylic acids such as formic acid, acetic acid, propionic acid, butyric acid, isobutyric acid, valeric acid, isovaleric acid and pivalic acid; ketones such as acetone, methyl ethyl ketone, methyl isobutyl ketone, diethyl ketone, dipropyl ketone, diisopropyl ketone and dibutyl ketone; dimethyl sulfoxide; and light water (water).

When deuteration is carried out using a catalyst selected from a palladium catalyst, a platinum catalyst, a rhodium catalyst, a ruthenium catalyst, a nickel catalyst and a cobalt catalyst, which are not subjected to activation processing (hereinafter, may be abbreviated as "catalyst relating to the present invention"), a gas phase in a deuteration reactor may be replaced by an inert gas such as nitrogen and argon.

In addition, in the deuteration method of the present invention, a reactor is preferably under a sealed or a nearly-sealed condition resulting in a pressurized condition of the reaction system. The nearly-sealed condition is applied to the reaction such as the so-called continuous reaction where a reaction substrate is continuously injected to a reactor and a reaction product is continuously taken out of the reactor.

In addition, when a reactor is under a sealed condition in deuteration method relating to the present invention, the reaction temperature can be easily raised leading to efficient deuteration.

When a catalyst relating to the present invention is used in the deuteration of sealed state, as hydrogen gas or heavy hydrogen gas is not used in the deuteration, the carbon-carbon double bond contained in a compound represented by the general formula [1] is hardly reduced and deuteration alone proceeds.

The catalyst relating to the present invention includes a palladium catalyst, a platinum catalyst, a rhodium catalyst, a ruthenium catalyst, a nickel catalyst and a cobalt catalyst, which are not subjected to activation processing as mentioned above, among these a rhodium catalyst is preferable. Each of these catalysts can be effectively used for the deuteration method of the present invention by itself or in combination with one another as appropriate. The preferable combination of these catalysts includes, for example, a mixed catalyst of a rhodium catalyst and a platinum catalyst and a mixed catalyst of a rhodium catalyst and a palladium catalyst.

The palladium catalyst includes one having usually 0 to 4, preferably 0 to 2 and more preferably 0 valence of a palladium atom.

The platinum catalyst includes one having usually 0 to 4, preferably 0 to 2 and more preferably 0 valence of a platinum atom.

The rhodium catalyst includes one having usually 0 or 1, preferably 0 valence of a rhodium atom.

The ruthenium catalyst includes one having usually 0 to 2, preferably 0 valence of a ruthenium atom.

The nickel catalyst includes one having usually 0 to 2, preferably 0 valence of a nickel atom.

The cobalt catalyst includes one having usually 0 or 1, preferably 1 valence of a cobalt atom.

The above catalyst may be a metal itself, a metal oxide, a metal halide, a metal acetate or a metal coordinated with a ligand, or may be a metal itself, a metal oxide, a metal halide, a metal acetate or a metal complex that is supported on various carriers.

Hereinafter, a catalyst supported on a carrier may be abbreviated as a "carrier-supported metal catalyst", and a catalyst not supported on a carrier may be abbreviated as a "metal catalyst".

The ligand of a metal catalyst which may be coordinated with a ligand among the catalysts relating to the deuteration method of the present invention includes, for example, 1,5-cyclooctadiene (COD), dibenzylidene acetone (DBA), bipyridine (BPY), phenanthroline (PHE), benzonitrile (PhCN), isocyanide (RNC), triethylarsine (As(Et)₃), acetylacetonate (acac); an organic phosphine ligand such as dimethylphenylphosphine (P(CH₃)₂Ph), diphenylphosphinoferrocene (DPPF), trimethylphosphine (P(CH₃)₃), triethylphosphine (PEt₃), tri-tert-butylphosphine (P^{t}Bᵤ₃), tricyclohexylphosphine (PCys), trimethoxyphosphine (P(OCH₃)₃), triethoxyphosphine (P(OEt)₃), tri-tert-butoxyphosphine (P(O^{t}Bu)3), triphenylphosphine (PPh₃), 1,2-bis(diphenylphosphino)ethane (DPPE), triphenoxyphosphine (P(OPh)₃) and tri-o-tolylphosphine (P(O-tolyl)₃).

Specific examples of the palladium based metal catalyst include, for example, Pd; palladium hydroxide catalysts such as Pd(OH)₂; palladium oxide catalysts such as PdO; halogenated palladium catalysts such as PdBr₂, PdCl₂ and PdI₂; palladium acetate catalysts such as palladium acetate (Pd(OAc)₂) and palladium trifluoroacetate (Pd(OCOCF₃)₂); and for example, palladium metal complex catalysts which are coordinated with a ligand such as Pd(RNC)₂Cl₂, Pd(acac)2, diacetate-bis-(triphenylphosphine)palladium [Pd(OAc)₂(PPh₃)₂], Pd(PPh₃)₄, Pd₂(dba)₃, Pd(NH₃)₂Cl₂, Pd(CH₃CN)₂Cl₂, dichloro-bis-(benzonitrile)palladium [Pd(PhCN)₂Cl₂], Pd(dppe)Cl₂, Pd(dppf)Cl_{2,} Pd(PCy₃)₂Cl₂, Pd(PPh₃)₂Cl₂, Pd[P(o-tolyl)₃]₂Cl₂, Pd(cod)₂Cl₂ and Pd(PPh₃)(CH₃CN)₂Cl₂.

Specific examples of the platinum based metal catalyst include, for example, Pt; platinum catalysts such as PtO₂, PtCl₄, PtCl₂ and K₂PtCl₄; platinum catalysts which are coordinated with a ligand such as PtCl₂(cod), PtCl₂(dba), PtCl₂(PCy₃)a, PtCl₂(P(OEt)₃)₂, PtCl₂(P(O^{t}Bu)₃)₂, PtCl₂(bpy), PtCl₂(phe), Pt(PPh₃)₄, Pt(cod)₂, Pt(dba)₂, Pt(bpy)₂ and Pt(phe)₂.

Specific examples of the rhodium based metal catalyst include, for example, Rh, rhodium catalysts which are coordinated with a ligand such as RhCl(PPh₃)₃.

Specific examples of the ruthenium based metal catalyst include, for example, Ru, ruthenium catalysts which are coordinated with a ligand such as RuCl₂(PPh₃)₃.

Specific examples of the nickel based metal catalyst include, for example, Ni; nickel catalysts such as NiCl₂ and NiO; nickel catalysts which are coordinated with a ligand such as NiCl₂(dppe), NiCl₂(PPh₃)₂, Ni(PPh₃)₄, Ni(P(OPh)₃)₄ and Ni(cod)₂.

Specific examples of the cobalt based metal catalyst include, for example, cobalt metal complex catalysts which are coordinated with a ligand such as CO(C₃H₅){P(OCH₃)₃}3.

The carrier, in the case where the above catalyst is supported on a carrier, includes, for example, carbon, alumina, silica gel, zeolite, molecular sieves, ion-exchange resins and polymers, and among these carbon is preferable.

The ion exchange resin used as a carrier may be a resin having no adverse effect on deuteration of the present invention, and includes, for example, a cation exchange resin and an anion exchange resin.

The cation exchange resin includes, for example, a weakly acidic cation exchange resin and a strongly acidic cation exchange resin. The anion exchange resin includes, for example, a weakly basic anion exchange resin and a strongly basic anion exchange resin.

The ion exchange resin generally contains a polymer cross-linked by a bifunctional monomer as a skeleton polymer to which an acidic group or a basic group is bonded, and then is exchanged by various cations or anions (counter ion), respectively.

Specific examples of the weakly acidic cation exchange resin include, for example, a resin obtained by hydrolysis of a polymer of an acrylic ester or a methacrylic ester cross-linked by divinylbenzene.

Specific examples of the strongly acidic cation exchange resin include, for example, a resin obtained by sulfonation of a styrene-divinylbenzene copolymer.

Specific examples of the strongly basic anion exchange resin include, for example, a resin obtained by bonding an amino group to an aromatic ring of a styrene-divinylbenzene copolymer.

Strength of basicity of a basic anion exchange resin increases with an amino group in the order of a primary amino group, a secondary amino group, a tertiary amino group and a quaternary ammonium salt.

An ion exchange resin available on the market may be used similarly to the above ion exchange resins as a carrier of a catalyst relating to deuteration of the present invention.

The polymer used as a carrier is not especially limited unless it has an adverse effect on deuteration of the present invention. An example of such polymers includes one obtained by polymerization or copolymerization of a monomer shown by the following general formula [3].

The general formula [3]:

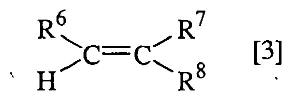

(wherein, R⁶ indicates a hydrogen atom, a lower alkyl group, a carboxyl group, a carboxyalkyl group, an alkoxycarbonyl group, a hydroxyalkoxycarbonyl group, a cyano group or a formyl group; R⁷ indicates a hydrogen atom, a lower alkyl group, a carboxyl group, an alkoxycarbonyl group, a hydroxyalkoxycarbonyl group, a cyano group or a halogen atom; R⁸ indicates a hydrogen atom, a lower alkyl group, a haloalkyl group, a hydroxyl group, an aryl group which may have a substituent, an aliphatic heterocyclic group, an aromatic heterocyclic group, a halogen atom, an alkoxycarbonyl group, a hydroxyalkoxycarbonyl group, a sulfo group, a cyano group, a cyano-containing alkyl group, an acyloxy group, a carboxyl group, a carboxyalkyl group, an aldehyde group, an amino group, an aminoalkyl group, a carbamoyl group, an N-alkylcarbamoyl group or a hydroxyalkyl group; and R⁶ and R⁷ may be bonded to form an alicyclic ring together with the adjacent - C=C - bond).

In the general formula [3], the lower alkyl group indicated by R⁶ to R⁸ may be straight chained, branched or cyclic, and includes, for example, an alkyl group having 1 to 6 carbon atoms and specifically a methyl group, an ethyl group, a n-propyl group, an isopropyl group, a n-butyl group, an isobutyl group, a sec-butyl group, a tert-butyl group, a n-pentyl group, an isopentyl group, a sec-pentyl group, a tert-pentyl group, a neopentyl group, a 1-methylpentyl group, a n-hexyl group, an isohexyl group, a sec-hexyl group, a tert-hexyl group, a neohexyl group, a cyclopropyl group, a cyclopentyl group and a cyclohexyl group.

The carboxyalkyl group indicated by R⁶ and R⁸ includes, for example, a group formed by replacing part of hydrogen atoms of the above lower alkyl group indicated by R⁶ to R⁸ with a carboxyl group and specifically, for example, a carboxymethyl group, a carboxyethyl group, a carboxypropyl group, a carboxybutyl group, a carboxypentyl group and a carboxyhexyl group.

The alkoxycarbonyl group indicated by R⁶ to R⁸ includes preferably, for example, one having 2 to 11 carbon atoms and specifically, for example, a methoxycarbonyl group, an ethoxycarbonyl group, a propoxycarbonyl group, a butoxycarbonyl group, a pentyloxycarbonyl group, a hexyloxycarbonyl group, a heptyloxycarbonyl group, an 2-ethylhexyloxycarbonyl group, an octyloxycarbonyl group, a nonyloxycarbonyl group and a decyloxycarbonyl group.

The hydroxyalkoxycarbonyl group indicated by R⁶ to R⁸ includes a group formed by replacing part of hydrogen atoms of the above alkoxycarbonyl group having 2 to 11 carbon atoms with a hydroxyl group and specifically, for example, a hydroxymethoxycarbonyl group, a hydroxyethoxycarbonyl group, a hydroxypropoxycarbonyl group, a hydroxybutoxycarbonyl group, a hydroxypentyloxycarbonyl group, a hydroxyhexyloxycarbonyl group, a hydroxyheptyloxycarbonyl group, a hydroxyoctyloxycarbonyl group, a hydroxynonyloxycarbonyl group and a hydroxydecyloxycarbonyl group.

The halogen atom indicated by R⁷ and R⁸ includes, for example, fluorine, chlorine, bromine and iodine.

The haloalkyl group indicated by R⁸ includes, for example, a group having 1 to 6 carbon atoms that is formed by halogenating (e.g., fluorinating, chlorinating, brominating, iodinating etc.) the above lower alkyl group indicated by R⁶ to R⁸ and specifically, for example, a fluoromethyl group, a chloromethyl group, a bromomethyl group, an iodomethyl group, a trifluoromethyl group, a trichloromethyl group, a tribromomethyl group, a triiodomethyl group, a 2-fluoroethyl group, a 2-chloroethyl group, a 2-bromoethyl group, an 2-iodoethyl group, a 3-fluoropropyl group, a 3-chloropropyl group, a 3-bromopropyl group, a 3,3,3-trifluoropropyl group, a 3,3,3-trichloropropyl group, a 3,3,3-tribromopropyl group, a 4-fluorobutyl group, a 4-chlorobutyl group, a 4-bromobutyl group, a 5-fluoropentyl group, a 5-chloropentyl group, a 5-bromopentyl group, a 6-fluorohexyl group, a 6-chlorohexyl group and a 6-bromohexyl group.

The aryl group of the aryl group which may have a substituent includes, for example, a phenyl group, a tolyl group, a xylyl group and a naphthyl group, and said substituent group includes, for example, an amino group; a hydroxyl group; an alkoxy group of 1 to 4 carbon atoms such as a methoxy group, an ethoxy group, a n-propoxy group, an isopropoxy group, a n-butoxy group, an isobutoxy group, a sec-butoxy group and a tert-butoxy group; and a carboxyl group. Specific examples of the substituted aryl group include, for example, an aminophenyl group, a toluidino group, a hydroxyphenyl group, a methoxyphenyl group, a tert-butoxyphenyl group and a carboxyphenyl group.

The aliphatic heterocyclic group includes, for example, a 5- or 6-membered ring having 1 to 3 hetero atoms such as a nitrogen atom, an oxygen atom and a sulfur atom, and specifically, for example, a 2-oxopyrrolidyl group, a piperidyl group, a piperidino group, a piperazinyl group and a morpholino group.

The aromatic heterocyclic group includes preferably, for example, a 5- or 6-membered ring having 1 to 3 hetero atoms such as a nitrogen atom, an oxygen atom and a sulfur atom, and specifically, for example, a pyridyl group, an imidazolyl group, a thiazolyl group, a furyl group and a pyranyl group.

The cyano-containing alkyl group includes, for example, a group formed by replacing part of hydrogen atoms of the above lower alkyl group indicated by R⁶ to R⁸ with cyano groups, and specifically, for example, a cyanomethyl group, a 2-cyanoethyl group, a 2-cyanopropyl group, a 3-cyanopropyl group, a 2-cyanobutyl group, a 4-cyanobutyl group, a 5-cyanopentyl group and a 6-cyanohexyl group.

The acyloxy group includes, for example, a group derived from a carboxylic acid having 2 to 20 carbon atoms and specifically, for example, an acetyloxy group, a propionyloxy group, a butyryloxy group, an isobutyryloxy group, a valeryloxy group, an isovaleryloxy group, a pivaloyloxy group, a hexanoyloxy group, a heptanoyloxy group, an octanoyloxy group, a nonanoyloxy group, a decanoyloxy group, an undecanoyloxy group, a lauroyloxy group, a tridecanoyloxy group, a myristoyloxy group, a pentadecanoyloxy group, a palmitoyloxy group, a heptadecanoyloxy group, a stearoyloxy group, a nonadecanoyloxy group, an icosanoyloxy group and a benzoyloxy group.

The aminoalkyl group includes a group formed by replacing part of hydrogen atoms of the above lower alkyl group indicated by R⁶ to R⁸ with amino groups, and specifically, for example, an aminomethyl group, an aminoethyl group, an aminopropyl group, an aminobutyl group, an aminopentyl group and an aminohexyl group.

The N-alkylcarbamoyl group includes a group formed by replacing part of hydrogen atoms of a carbamoyl group with alkyl groups having 1 to 6 carbon atoms, and specifically, for example, an N-methylcarbamoyl group, an N-ethylcarbamoyl group, an N-n-propylcarbamoyl group, an N-isopropylcarbamoyl group, an N-n-butylcarbamoyl group and an N-tert-butylcarbamoyl group.

The hydroxyalkyl group includes a group formed by replacing part of hydrogen atoms of the above lower alkyl group indicated by R⁶ to R⁸ with hydroxyl groups, and specifically, for example, a hydroxymethyl group, a hydroxyethyl group, a hydroxypropyl group, a hydroxybutyl group, a hydroxypentyl group and a hydroxyhexyl group.

The alicyclic ring in the case where R⁶ and R⁷ are bonded together with the adjacent - C=C - bond to form an alicyclic ring, may be monocyclic or polycyclic and includes, for example, an unsaturated alicyclic ring having 5 to 10 carbon atoms, and specifically, for example, a norbornene ring, a cyclopentene ring, a cyclohexene ring, a cyclooctene ring and a cyclodecene ring.

The specific examples of the monomer represented by the general formula [3] include ethylenically unsaturated aliphatic hydrocarbons having 2 to 20 carbon atoms such as ethylene, propylene, butylene and isobutylene; ethylenically unsaturated aromatic hydrocarbons having 8 to 20 carbon atoms such as styrene, 4-methylstyrene, 4-ethylstyrene and divinylbenzene; alkenyl esters having 3 to 20 carbon atoms such as vinyl formate, vinyl acetate, vinyl propionate and isopropenyl acetate; halogen-containing ethylenically unsaturated compounds having 2 to 20 carbon atoms such as vinyl chloride, vinylidene chloride, vinylidene fluoride and tetrafluoroethylene; ethylenically unsaturated carboxylic acids having 3 to 20 carbon atoms such as acrylic acid, methacrylic acid, itaconic acid, maleic acid, fumaric acid, crotonic acid, vinylacetic acid, allylacetic acid and vinylbenzoic acid (These acids may take a form of a salt of an alkali metal such as sodium and potassium or an ammonium salt.); ethylenically unsaturated carboxylic esters such as methyl methacrylate, ethyl methacrylate, propyl methacrylate, butyl methacrylate, 2-ethylhexyl methacrylate, methyl acrylate, ethyl acrylate, propyl acrylate, butyl acrylate, 2-ethylhexyl acrylate, lauryl methacrylate, stearyl acrylate, methyl itaconate, ethyl itaconate, methyl maleate, ethyl maleate, methyl fumarate, ethyl fumarate, methyl crotonate, ethyl crotonate and methyl 3-butenoate; cyano-containing ethylenically unsaturated compounds having 3 to 20 carbon atoms such as acrylonitrile, methacrylonitrile and allyl cyanide; ethylenically unsaturated amide compounds having 3 to 20 carbon atoms such as acrylamide and methacrylamide; ethylenically unsaturated aldehydes having 3 to 20 carbon atoms such as acrolein and crotonaldehyde; ethylenically unsaturated sulfonic acids having 2 to 20 carbon atoms such as vinylsulfonic acid and 4-vinylbenzene sulfonic acid (These acids may take a form of a salt of an alkali metal such as sodium and potassium.); ethylenically unsaturated aliphatic amines having 2 to 20 carbon atoms such as vinylamine and allylamine; ethylenicically unsaturated aromatic amines having 8 to 20 carbon atoms such as vinylaniline; ethylenically unsaturated aliphatic heterocyclic amines having 5 to 20 carbon atoms such as N-vinylpyrrolidone and vinylpiperidine; ethylenically unsaturated alcohols having 3 to 20 carbon atoms such as allyl alcohol and crotyl alcohol; ethylenically unsaturated phenols having 8 to 20 carbon atoms such as 4-vinylphenol.

When the above polymers are used as a carrier, a carrier that is hardly deuterated itself in deuteration of the present invention is preferably used. However, a catalyst supported on a deuteratable carrier itself can also be used for deuteration of the present invention.

In the deuteration method of the present invention, a carrier-supported rhodium catalyst is preferably used among various carrier-supported catalysts and specifically a rhodium carbon is particularly preferable.

In the carrier-supported catalyst, the content of the catalyst metal, that is, palladium, platinum, rhodium, ruthenium, nickel or cobalt, is usually 1 to 99% by weight, preferably 1 to 50% by weight, more preferably 1 to 30% by weight, still more preferably 1 to 20% by weight, and particularly preferably 5 to 10% by weight, based on the total amount of the catalyst.

In the deuteration method of the present invention, the amount of a catalyst to be used, relating to the present invention is usually the so-called catalyst amount, preferably in the order of, 0.01 to 200% by weight, 0.01 to 100% by weight, 0.01 to 50% by weight, 0.01 to 20% by weight, 0.1 to 20% by weight, 1 to 20% by weight and 10 to 20% by weight, based on a compound represented by the general formula [1] to be used as a reaction substrate regardless of whether the catalyst is supported by a carrier or not, and the upper limit content of the catalyst metal in said whole catalyst is preferably in the order of, 20% by weight, 10% by weight, 5% by weight and 2% by weight, whereas the lower limit content is preferably in the order of, 0.0005% by weight, 0.005% by weight, 0.05% by weight and 0.5% by weight.

In deuteration of a compound represented by the above general formula [1], a combined catalyst of 2 or more catalysts among the above various catalysts can be used and sometimes serves to improve a deuteration ratio. Such a combination of catalysts for deuterating a compound represented by the general formula [1] includes, for example, a combination of a rhodium catalyst and a proper catalyst such as a combination of a palladium catalyst and a rhodium catalyst, a combination of a platinum catalyst and a rhodium catalyst and a combination of a ruthenium catalyst and a rhodium catalyst. Among these combinations, a combination of a platinum catalyst and a rhodium catalyst is preferable, and one or both of them may be supported by a carrier. Preferable specific examples include a combination of a platinum carbon and a rhodium carbon.

The amount of the catalysts to be used in the combination of 2 or more catalysts may be set so that the total amount of the catalysts in the combination may be matched with the above mentioned amount of a catalyst. The ratio of the amount of each catalyst to be used is not particularly limited. For example, in the case where the above combination of a platinum carbon and a rhodium carbon is used, the weight of platinum in the catalyst may be set usually 0.01 to 100 times, preferably 0.1 to 10 times and more preferably 0.2 to 5 times, relative to the weight of rhodium.

Reaction temperature in the deuteration method of the present invention, the lower limit is usually 10°C, preferably in the order of 20°C, 40°C, 60°C, 80°C, 110°C, 140°C and 160°C, whereas the upper limit is usually 300°C, preferably in the order of 200°C and 180°C.

Reaction time in the deuteration method of the present invention is usually 30 minutes to 72 hours, preferably 1 to 48 hours, more preferably 3 to 30 hours and still more preferably 6 to 24 hours.

In addition, when a carbon-carbon double bond contained in a compound represented by the general formula [1] tends to polymerize in the deuteration reaction of the present invention, for example, a polymerization inhibitor may be added to the reaction system of deuteration to suppress the polymerization.

The deuteration method of the present invention is described in detail as follows.

The deuteration method of the present invention using a catalyst relating to the present invention is specifically described taking as an example the case to use deuterated water as a heavy hydrogen source and use a rhodium carbon (Rh/C) (Rh content: 5%) as a catalyst.

Namely, for example, 1 mole of a compound (substrate) represented by the general formula [1] and Rh/C (prepared by an ordinary method and not subjected to activation processing after preparation) of 0.01 1 to 200% by weight based on the substrate are added to deuterated water the amount of which is enough to contain 10 to 150 molar times of heavy hydrogen atoms based on the deuteratable hydrogen atoms of the substrate. After the reactor is sealed and has its gas phase replaced by an inert gas, reaction is carried out at about 110 to 200°C in an oil bath for about 1 to 48 hours under stirring to yield a deuterated compound of the compound represented by the general formula [1].

After termination of the reaction, when an obtained reaction product is soluble in a deuterated solvent, a catalyst is removed by filtering the reaction mixture. After the filtrate is concentrated, the product is isolated and subjected to structural analysis by measuring its ¹H-NMR, ²H-NMR and mass spectrum. When an obtained reaction product is hardly soluble in a deuterated solvent, the reaction product is isolated and then subjected to structural analysis by measuring its ¹H-NMR, ²H-NMR and mass spectrum.

When it is difficult to isolate a product from a reaction mixture, the product may be subjected to structural analysis by measuring ¹H-NMR of the filtrate as it is, using a proper standard substance. When an obtained reaction product is hardly soluble in a deuterated solvent, the product can be isolated from the reaction mixture according to, for example, a known purification method where the product is extracted from the reaction mixture with an organic solvent in which the product is soluble and then a catalyst is removed by filtration.

When X in a compound (substrate) represented by the general formula [1] is a chlorine atom and the catalyst relating to the present invention is a catalyst containing a rhodium catalyst not subjected to activation processing such as being brought into contact with hydrogen gas or heavy hydrogen gas, the deuteration method of the present invention hardly produces impurities such as a substance formed by reduction of a carbon - carbon double bond in the substrate by hydrogenation and a substance formed by reductive elimination of the chlorine and thus can provide an object product at a high deuteration ratio. On the other hand, when a catalyst subjected to activation processing as mentioned above is used, such impurities as mentioned above are formed and thus the product can not obtained at a high deuteration ratio.

Among deuterated compounds represented by the general formula [1], the compound of which R³ in the general formula [1] is a light hydrogen atom or a heavy hydrogen atom, easily gives a compound of which R³ is a light hydrogen atom when processed with, for example, light water (water) after the above deuteration reaction, and a compound of which R³ is a heavy hydrogen atom when processed with, for example, deuterated water (D₂O) after the above deuteration reaction.

Thus obtained deuterated compound represented by the general formula [1] is represented by the general formula [2]:

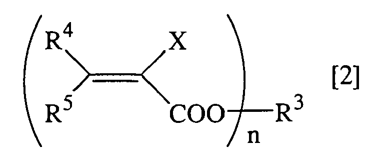

(wherein, R⁴ and R⁵ indicate each independently a light hydrogen atom or a heavy hydrogen atom and at least one of R⁴ and R⁵ indicates a heavy hydrogen atom; n indicates 1 or 2; and R³ and X are the same as the above).

At least one of R⁴ and R⁵ in the general formula [2] may be a heavy hydrogen atom, but preferably both of them are heavy hydrogen atoms.

Specific examples of the deuterated compounds represented by the general formula [2] include a compound formed by deuterating at least one deuteratable hydrogen atom in a compound represented by the general formula [1], and specifically for example, deuterated haloacrylic acids such as 2-chloroacrylic acid-d (DHC=C(Cl)COOH), 2-chloroacrylic acid-d₂ (D₂C=C(Cl)COOH, DHC=C(Cl)COOD), 2-chloroacrylic acid-d₃ (D₂C= C(Cl)COOD), 2-fluoroacrylic acid-d (DHC=CFCOOH), 2-fluoroacrylic acid-d₂ (D₂C=CFCOOH, DHC=CFCOOD), 2-fluoroacrylic acid-d₃ ((D₂C= CFCOOD), 2-bromoacrylic acid-d (DHC=C(Br)COOH), 2-bromoacrylic acid-d₂ (D₂C=C(Br)COOH, DHC=C(Br)COOD), 2-bromoacrylic acid-d₃ (D₂C=C(Br)COOD), 2-iodoacrylic acid-d (DHC=CICOOH), 2-iodoacrylic acid-d₂ (D₂C=CICOOH, DHC=CICOOD), 2-iodoacrylic acid-d₃ (D₂C= CICOOD); and salts of these acids (e.g., alkaline metal salt such as sodium salt, potassium salt, rubidium salt and cesium salt; and alkaline earth metal salt such as magnesium salt, calcium salt, strontium salt and barium salt), among these, for example, 2-chloroacrylic acid-d₂ (D₂C=C(Cl)COOH), 2-chloroacrylic acid-d₃, sodium 2-chloroacrylate-d₂, sodium 2-fluoroacrylate-d₂ and sodium 2-bromoacrylate-d₂ are preferable, in particular, for example, sodium 2-chloroacrylate-d₂, 2-chloroacrylic acid-d₂ (D₂C=C(Cl)COOH) and 2-chloroacrylic acid-d₃ are more preferable.

To obtain a deuterated compound represented by the above general formula [2] (hereinafter, may be abbreviated as a "deuterated compound relating to the present invention"), either a conventional deuteration method or the deuteration method of the present invention may be used to deuterate a compound represented by the general formula [1]. The deuteration method of the present invention, however, gives more efficiently a deuterated compound having a higher deuteration ratio.

The deuteration method of the present invention uses a catalyst not subjected to activation processing such as being brought into contact with hydrogen gas or heavy hydrogen gas after prepared by an ordinary method (hereinafter, may be abbreviated as "non-activated catalyst relating to the present invention"), and thus can efficiently produce the deuterated compound having a high deuteration ratio without causing the problem that impurities such as a substance formed by reduction of a carbon - carbon double bond in a compound represented by the general formula [1] by hydrogenation and a substance formed by reductive elimination of a halogen in the compound are produced as by-products.

In addition, when the deuteration of a compound represented by the general formula [1] is carried out by using a conventional method, for example, the impurities are produced as by-product. And it is sometimes difficult to separate formed impurities and the product deuterated compound. In such a case, the deuteration method of the present invention hardly forms impurities as by-product and thus can efficiently produce the object deuterated compound at a high deuteration ratio without any need of separating the impurities and the deuterated compound.

Further, among the deuteration method of the present invention, when a catalyst such as a rhodium carbon catalyst, a mixed catalyst of a rhodium carbon and a platinum carbon and a mixed catalyst of a rhodium carbon and a palladium carbon is used, or when the above catalyst which is not subjected to activation processing such as being brought into contact with hydrogen gas or heavy hydrogen gas is used, a deuterated compound represented by the general formula [2] can be easily obtained having a deuteration ratio of usually 50% or more, preferably in the order of 60% or more, 70% or more, 75% or more, 80% or more, 85% or more and 90% or more.

Thus obtained deuterated compound is a very useful compound as, for example, an intermediate of a monomer for an optical fiber.

As mentioned above, the deuteration method of the present invention using a catalyst relating to the present invention and using a deuterated solvent as a heavy hydrogen source provides a deuterated compound having a high deuteration ratio nearly without reducing a carbon-carbon double bond in a compound represented by the general formula [1] by hydrogenation and causing reductive elimination of a halogen.

As mentioned above, the deuteration method of the present invention in which a compound represented by the general formula [1] is reacted with a heavy hydrogen source in the presence of a catalyst not subjected to activation processing can efficiently deuterate (deuterate and tritiate) the compound nearly without reducing a carbon-carbon double bond in the compound and causing reductive elimination of a halogen in the compound.

In addition, the deuteration method of the present invention does not require an acidic or basic condition in deuteration reaction and thus not only improves work environment, but also can be applied to deuteration of a substrate that tends to decompose under a high-temperature, acidic or basic condition.

The present invention will be described more specifically with the following examples and comparative examples, without being limited thereto at all.

### Examples

The catalyst used in the examples is a catalyst not subjected to activation processing after prepared by an ordinary method. A palladium carbon (Pd/C) containing 10% of Pd, a platinum carbon (Pt/C) containing 5% of Pt and a rhodium carbon (Rh/C) containing 5% of Rh were used. In examples, "non-active" means "not subjected to activation processing".

### Example 1. Deuteration of sodium 2-chloroacrylate (catalyst: Rh/C)

Sodium 2-chloroacrylate of 500 mg and a rhodium carbon (Rh: 5%) of 100 mg were suspended in deuterated water of 17 mL, and reacted at 160°C in an oil bath for about 24 hours after replacing the reaction system with nitrogen. After termination of the reaction, the reaction mixture was filtered to remove the catalyst and concentrated under reduced pressure. The obtained compound was then subjected to structural analysis by measuring its ¹H-NMR and ²H-NMR to show an isolation yield of 76% and a deuteration ratio of 95% of sodium 2-chloroacrylate. The results are shown in Table 1.

### Examples 2 to 3

Sodium 2-chloroacrylate was subjected to deuteration reaction similarly as in Example 1 except for using non-active catalysts (mixed - catalysts) shown in the following Table 1. Isolation yield and deuteration ratio of the obtained products are shown together in Table 1.

**Table 1**

| Exam. | substrate | catalyst | isolation yield (%) | deuteration ratio (%) |
|---|---|---|---|---|
| 1 | Sodium 2-chloroacrylate | Non-active 5%Rh/C 100 mg | 76 | 95 |
| 2 | Sodium 2-chloroacrylate | Non-active 5%Rh/C 100 mg | 86 | 96 |
| | | Non-active 5%Pt/C 100 mg | | |
| 3 | Sodium 2-chloroacrylate | Non-active 5%Rh/C 100 mg | 76 | 93 |
| | | Non-active 10%Pd/C 100 mg | | |

### Comparative Example 1. Deuteration of sodium acrylate (catalyst: Rh/C)

Deuteration reaction was carried out similarly as in Example 1 except for using sodium acrylate as a substrate to yield an isolation yield of 99% and a deuteration ratio of 46% of the sodium acrylate.

As is apparent from the results of Examples 1 to 3, it can be understood that the deuteration method of the present invention can efficiently deuterate a 2-haloacrylic acid and salt thereof.

As is clear from the results of Examples 2 and 3, it can be understood that the deuteration method of the present invention can efficiently deuterate a sodium haloacrylate even using a combined catalyst.

As apparent from the results of Examples 1 to 3, it can be founded that in the case where sodium 2-chloroacrylate is used as a substrate (that is, X in the general formula [1] is a chlorine atom), a rhodium catalyst (especially a rhodium carbon) is preferably used as a catalyst.

As apparent from the results of Examples 1 and Comparative Example 1, it can be understood that sodium acrylate as a substrate (Comparative Example 1) gives a low deuteration ratio, whereas a sodium haloacrylate, especially sodium 2-chloroacrylate, as a substrate (Example 1) gives the deuterated compound having a high deuteration ratio.

In addition, when deuterating a sodium haloacrylate (substrate) in the presence of a catalyst subjected to activation processing such as being brought into contact with hydrogen gas or heavy hydrogen gas, the deuteration ratio of the obtained deuterated compound turned out to be low probably because of reductive elimination of a halogen atom in the compound and reduction of a carbon - carbon double bond in the compound by hydrogenation.

## Claims

1. A deuteration method of a compound represented by the general formula [1]: 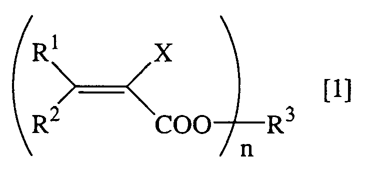 wherein, R¹ and R² indicate.each independently a light hydrogen atom or a heavy hydrogen atom and at least one of R¹ and R² indicates a light hydrogen atom; R³ indicates a light hydrogen atom, a heavy hydrogen atom, an alkaline metal atom or an alkaline earth metal atom; X indicates a halogen atom; and n indicates 1 or 2,
which comprises reacting the compound represented by the general formula [1] with a heavy hydrogen source in the presence of a catalyst selected from a palladium catalyst, a platinum catalyst, a rhodium catalyst, a ruthenium catalyst, a nickel catalyst and a cobalt catalyst that are not subjected to activation processing.

2. The deuteration method according to claim 1, wherein R³ indicates an alkyl metal atom in the general formula [1].

3. The deuteration method according to claim 1, wherein R³ indicates a sodium atom in the general formula [1].

4. The deuteration method according to claim 1, wherein X indicates a chlorine atom in the general formula [1].

5. The deuteration method according to claim 1, wherein both of R¹ and R² indicate a light hydrogen atom in the general formula [1].

6. The deuteration method according to claim 1, wherein the heavy hydrogen source is a deuterated solvent.

7. The deuteration method according to claim 6, wherein the deuterated solvent is deuterated water (D₂O).

8. The deuteration method according to claim 1, wherein the catalyst selected from a palladium catalyst, a platinum catalyst, a rhodium catalyst, a ruthenium catalyst, a nickel catalyst and a cobalt catalyst is a catalyst containing a rhodium catalyst.

9. The deuteration method according to claim 8, wherein the catalyst containing a rhodium catalyst is a rhodium catalyst, a mixed catalyst of a rhodium catalyst and a palladium catalyst, and a mixed catalyst of a rhodium catalyst and a platinum catalyst.

10. The deuteration method according to claim 8, wherein the rhodium catalyst is a rhodium carbon.

11. The deuteration method according to claim 1, wherein the compound represented by the general formula [1] is sodium 2-chloroacrylate; and the catalyst selected from a palladium catalyst, a platinum catalyst, a rhodium catalyst, a ruthenium catalyst, a nickel catalyst and a cobalt catalyst is a rhodium carbon.

12. A deuterated compound represented by the general formula [2]: 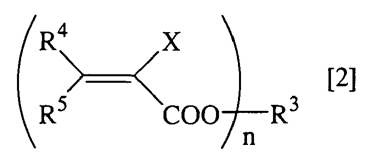 wherein, R⁴ and R⁵ indicate each independently a light hydrogen atom or a heavy hydrogen atom and at least one of R⁴ and R⁵ indicates a heavy hydrogen atom; R³ indicates a light hydrogen atom, a heavy hydrogen atom, an alkaline metal atom or an alkaline earth metal atom; X indicates a halogen atom; and n indicates 1 or 2.

13. The deuterated compound according to claim 12, wherein the deuteration ratio of the compound is 50% or higher.

14. The deuterated compound according to claim 12, wherein the compound represented by the general formula [2] is sodium 2-chloroacrylate-d₂.
